# EUROPEAN PATENT SPECIFICATION

(11) **EP 2 305 185 B1**
(45) Date of publication and mention of the grant of the patent: **03.10.2012**
(21) Application number: 10014958.2
(22) Date of filing: 23.08.2006
(51) Int. Cl.: A61F 5/441

(54) **Filter assembly for an ostomy pouch**
Filteranordnung für einen Ostomiebeutel
Assemblage de filtre pour un sac de stomie

(30) Priority: 23.08.2005 US 711193 P
(43) Date of publication of application: 06.04.2011
(62) Divisional of application: 06017515.5
(73) Proprietor: Bristol-Myers Squibb Company, New York, NY 10154 (US)
(72) Inventor: Suehr, Susan Lynn, Hillsborough, NJ 08844 (US); Oberholtzer, Gary E., Feasterville, PA 19053 (US)
(74) Representative: Mays, Julie

(56) References cited:
- EP-A- 0 443 728
- EP-A- 1 514 528
- GB-A- 2 287 193
- US-A- 3 952 727

## Description

### FIELD OF THE INVENTION

This invention is directed to a filter assembly and more particularly to a filter assembly for an ostomy pouch.

### BACKGROUND OF THE INVENTION.

The surgical creation of an ostomy is the therapy for many sufferers of diseases or injury of the gastrointestinal or urinary tract. An ostomy is a rerouting of the tract to an opening, or stoma, in the abdominal wall to permit excretion of bodily waste through the stoma to the outside of the patient's body. Once this opening has been created, the patient typically uses a device attached to their body by some means to collect and contain the bodily waste.

The waste is typically collected in a disposable ostomy pouch that is attached to the patient's peristomal area and around the stoma by means of an adhesive. Flatus or gases emitted from a stoma into an ostomy pouch, and gases that issue from waste material confined in an ostomy pouch, are usually evacuated from the pouch through a deodorizing filter so as to avoid potential embarrassment to the ostomate from the emission of an unpleasant odor. A gas outlet is provided adjacent to the deodorizing filter to ensure that the outward flow of gas passes through the filter before leaving the pouch.

Most ostomy pouches can be wom for a few days before the deodorizing capability of the filter begins to lose effectiveness. If the deodorizing filter is contaminated by contact with waste material, it may be desirable to replace the disposable pouch immediately due to loss of airflow or deodorizing action. Waste material contact can occur as a result of physical activity by the wearer that shifts the contents of the bag toward the deodorizing filter, especially if the waste material is a liquid or semi-liquid consistency.

Ostomy pouch filters are typically made of a porous medium, such as open cell foam that has been impregnated with particles of deodorizing material, such as activated carbon. Such filters function well enough to deodorize the gaseous output from a typical ostomate for up to 2-3 days. However, the useful lives of these filters are usually far shorter due to fouling by contact with stool and/or the liquid that is contained in stool. Once a portion of a filter becomes wetted, that portion of the filter usually ceases to function. In addition, the presence of stool on the filter or in the flow path leading to the filter can cause partial or total blockage of the filter. Finally, filters can become wetted by exposure to water from outside the pouch, as can occur when the wearer bathes, showers, or swims.

Previous filter designs have employed microporous membranes that permit flow of gases into the filter but prevent the passage of stool or liquid through the membranes and into the filter. However, in practice, membranes that are capable of protecting a filter for up to 5 days of wear have limited gas flow properties, i.e. 30 mbar back pressure @ 500 cc/min. However, membranes that allow significantly higher gas flow are generally only able to protect the filter against water intrusion for approximately 1-2 days. Intrusion by water is an indicator of membrane performance.

Resistance to intrusion of mineral oil is another important indicator of membrane performance in ostomy applications. Other commercially available membranes either leak before 5 days with mineral oil, or exhibit high backpressure, ranging from 30 to 60 mbar, at a gas flow rate of 500 cc/min.

Ostomy pouch filters are typically assembled as component parts directly into ostomy pouches during final assembly of the device. Furthermore, the construction of such filters typically differs from one ostomy product to another. Many of those designs employ using a membrane inside the pouch in order to protect the filter from getting wet.

One of the problems with such membranes is that they are made with support members that are to be used for welding into the pouch. Pouch designs that have exposed edges of the membrane to the stoma may cause irritation of the stoma and discomfort to the patient when in place on the patient's abdomen. This occurs when such membranes are welded facing the stoma.

EP-A-0 443 728 discloses a filter assembly according to the preamble of claim 1.

It is therefore an object of this invention to provide a filter assembly system for an ostomy pouch wherein the filter is protected from exposure to moisture and stool by means of a microporous membrane that can be welded to the pouch.

It is a further object of this invention to provide a filter assembly system for an ostomy pouch wherein the filter is protected from exposure to moisture and stool by means of a microporous membrane that can be welded to the pouch in front of the stoma in a manner so as not to expose its edges to the stoma.

It is also the object of this invention to provide a filter assembly having a membrane welded to the inside of the pouch in a manner so as to insure complete sealing of such membrane to the pouch and provide superior leak properties while still providing superior gas flow and deodorization capacity.

### SUMMARY OF THE INVENTION

A filter assembly for attachment to an ostomy pouch, the ostomy pouch being formed from a bodyside panel and a non-bodyside panel joined together, comprising:
a. a porous filter containing deodorizing material for deodorizing gas passing therethrough, said filter being attached to the non-bodyside panel, the non-bodyside panel having an outlet for outletting deodorizing gas therethrough to the outside of the pouch;
b. a composite two sided microporous membrane at least partly covering said filter for preventing ingress of liquids into the filter, said membrane being comprised of an expanded polytetrafluoroethylene layer on one side and a support layer of hydrophobic material on the other side, said membrane at least partly overlying said filter with said support layer facing said filter; and
c. a connecting film having inner and outer ends, said connecting film being welded at its inner end to said polytetrafluoroethylene layer and its outer end to said non-bodyside panel.

The deodorizing material is preferably activated carbon. The microporous membrane has ePTFE (expanded polytetrafluoroethylene) on one side and polyester (PTE) on the other. The membrane is preferably a laminate of these two materials.

The ePTFE side of the membrane is welded to a connecting film, and the connecting film is welded to the pouch film. The polyester side of the membrane faces the filter and preferably at least partly overlies the filter. The filter is attached to the pouch film preferably by welding. The gas in the pouch passes sequentially through the composite microporous membrane, filter and outlet to the outside of the pouch.

### BRIEF DESCRIPTION OF THE DRAWING

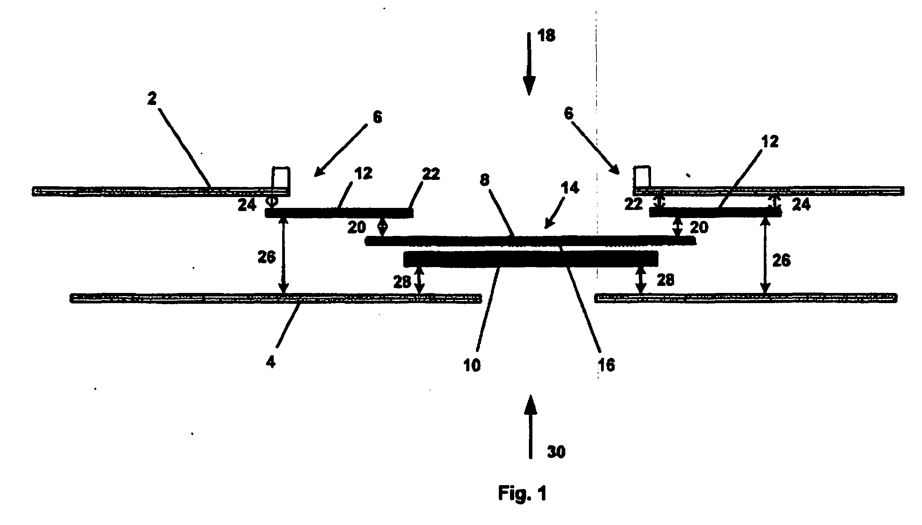

Fig. 1 is a schematic showing the layers of the filter assembly of the present invention.

### DETAILED DESCRIPTION OF THE INVENTION

The present invention is a filter assembly for an ostomy pouch. The ostomy pouch includes a bodyside film or panel 2 and a non-bodyside film or panel 4 welded together to form the pouch. The films 2 and 4 are preferably multilayered EVA (ethylene vinyl acetate) films commonly used in ostomy pouches. The filter assembly is positioned inside the pouch on the non-bodyside film 4. The bodyside film 2 is shown with a coupling 6 for attachment to the wearer's body directly or indirectly, as a one piece or a two piece ostomy system, respectively. In a one piece system, the pouch would be attached directly to the body with an adhesive. In a two piece system, the pouch would be coupled to a wafer that is adhesively attached to the wearer's body.

The filter assembly includes a composite microporous membrane 8, a filter 10 and a connecting film 12 for joining the composite membrane to the inside of the pouch on the non-bodyside film 4.

The composite microporous membrane 8 is comprised of two layers of materials: a) an expanded polytetrafluoroethylene (ePTFE) layer 14, and b) a hydrophobic support layer 16, preferably PET (polyester). The composite microporous 8 membrane is preferably a laminate and preferably has a thickness of 1.5 to 3.5 micron. The ePTFE layer 14 is exposed to the inside of the pouch and permits gases to pass through it.

A connecting film 12, preferably a multilayered EVA film commonly used in ostomy pouches, is welded at 20 near its inner edge or end 22 onto the ePTFE membrane surface and on the outer edge or end 24. It is welded onto the non-bodyside pouch film 4 at 26. The connecting film 12 is preferably greater than 75 micron film in thickness, for example 100 micron film, in order to allow complete sealing of the composite microporous membrane 8 at high temperatures and pressures during the welding process. The connecting film 12 may be the same used as the pouch film. The composite microporous membrane 8 is shown welded to the inside of the pouch directly in front of the stoma opening. The lack of edges to the filter assembly as a result of the manner in which the composite microporous membrane 8 is attached to the non-bodyside pouch film 4 reduces or eliminates irritation to the stoma by the filter assembly.

The hydrophobic PET support layer 16 of the composite microporous membrane 8 allows for long periods of exposure to water without leakage. Many other commercially available membranes employ hydrophilic support members that are able to prevent water penetration for only limited periods of time. Such membranes may display high initial resistance to water intrusion when tested, but do not prevent leakage of water components for the desired 5-day wear period.

Different types of filters can be used, for example, a round filter in which the gas enters the filter along its outer edge and exits the filter at the center of the external face of the filter, or a filter may have an axial flow path. The filter is attached to the non-bodyside film 4, preferably by welding at 28. An outlet 30 that includes at least one opening 32 extends through the non-bodyside film 4 to permit gas to pass from inside the pouch through the deodorizing filter 10 and outlet 30 to the outside of the pouch.

The composite microporous membrane 8 is preferably positioned so as to at least partly overlie the filter 10. The hydrophobic PET layer 16 faces the filter 10. The sealing of the composite microporous membrane 8 to the non-bodyside pouch film 4 protects the filter 10 from direct contact with the liquid or solid waste material in the pouch. The composite microporous membrane 8 protects the filter 10 and regulates the air flow through the filter 10, thus optimizing gas flow, deodorization time and leak resistance.

This invention has been put to practice in prototypes and demonstrated to have leak resistance of greater than 5 days with mineral oil, deodorization times of 70 to 90 minutes, and gas flow rates of 5 mbar backpressure at 500 cc/min. When subjected to water component fluids for 5 days it does not leak.

Other commercial filters on the market either leak before 5 days with mineral oil, or have low gas flow rates ranging from 30 to more than 60 mbar at 500 cc/min. A chart of how other filters compared in sample tests to an example of the current invention are shown below:

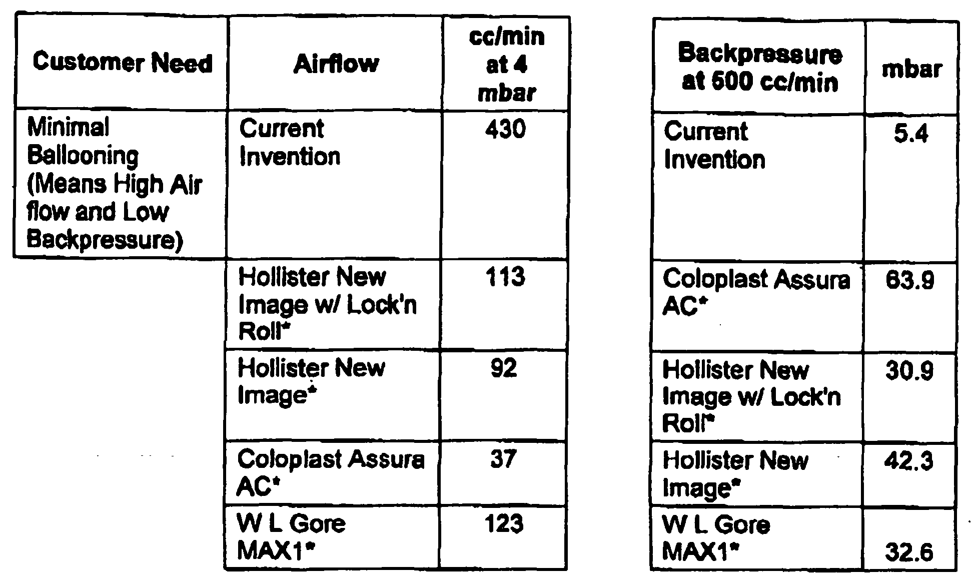

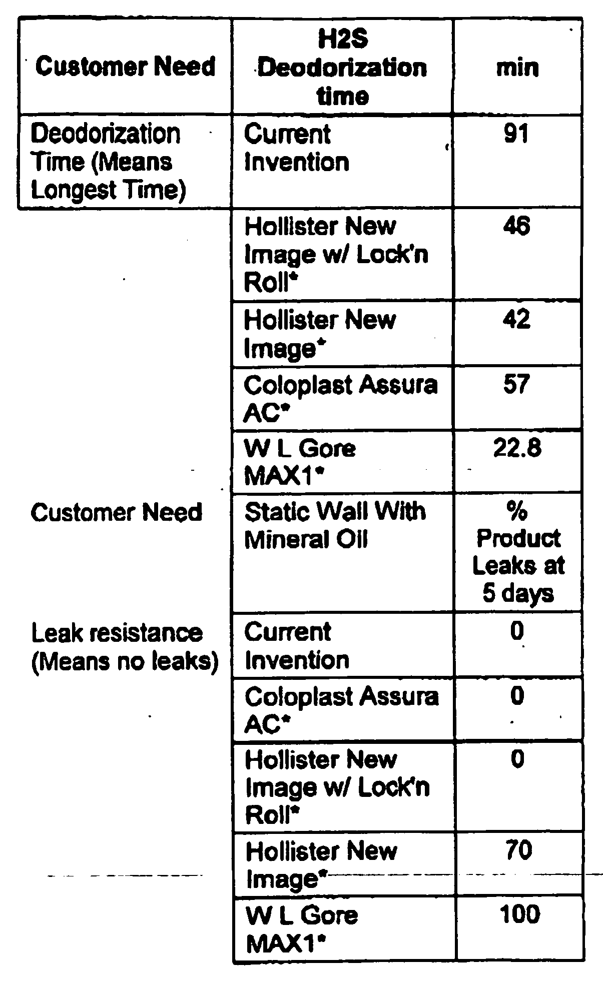

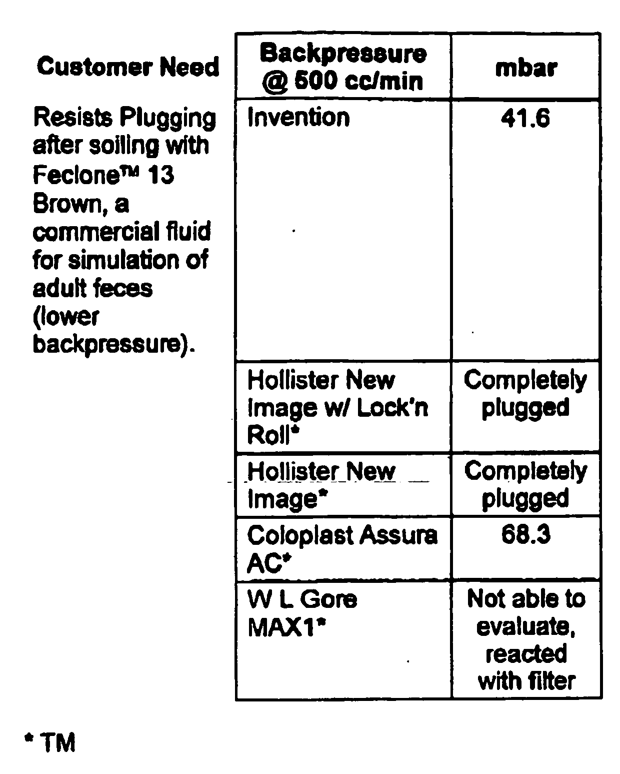

It will be appreciated that many modifications and alterations may be used without departing from the principles of the invention. Accordingly, the appended claims are intended to be broadly construed to include all such modifications and alternatives.

## Claims

1. A filter assembly for attachment to an ostomy pouch, the ostomy pouch being formed formed from a bodyside panel and a non-bodyside panel joined together, comprising:
a. a porous filter (10) containing deodorizing material for deodorizing gas passing therethrough, said filter being attached to the non-bodyside panel (4), the non-bodyside panel having an outlet (30) for outletting deodorizing gas therethrough to the outside of the pouch,
b. a composite two sided microporous membrane (8) at least partly covering said filter for obstructing ingress of liquids into the filter, said membrane being comprised of an expanded polytetrafluoroethylene layer (14) on one side and a support layer (16) of hydrophobic material on the other side, said membrane at least partly overlying said filter with said support layer facing said filter, and **characterised by**
c. a connecting film (12) having inner and outer ends, said connecting film (12) being welded at its inner end to said polytetrafluoroethylene layer (14) and its outer end to said non-bodyside panel (4).

2. The filter assembly as claimed in claim 1 wherein said support layer is PET (Polyester).

3. The filter assembly as claimed in claim 1 or 2 wherein said film welded to the membrane layer is 75 microns or more in thickness and has thermal plastic properties suitable for high temperature and pressure welding.

4. The filter assembly as claimed in claim 1, 2 or 3 wherein said deodorizing material is activated carbon.

5. The filter assembly of any preceding claim wherein said composite microporous membrane is a laminate.

6. The filter assembly as claimed in any preceding claim wherein said filter is welded to the non-bodyside panel on the inside of the ostomy pouch.

## Patentansprüche

1. Filteranordnung zur Befestigung an einem Stomabeutel, wobei der Stomabeutel aus einem körperseitigen Element und einem nichtkörperseitigen Element gebildet ist, die zusammengefügt sind, mit:
a. einem geruchsbeseitigendes Material enthaltenden porösen Filter (10) zur Geruchsbeseitigung von Gas, das es durchläuft, wobei das Filter am nichtkörperseitigen Element (4) befestigt ist und das nichtkörperseitige Element einen Auslass (30) zum Durchlassen von geruchsbeseitigendem Gas aus dem Beutel nach außen hat,
b. einer das Filter mindestens teilweise bedeckenden, zweiseitigen mikroporösen Verbundmembran (8) zum Versperren des Eindringens von Flüssigkeiten in das Filter, wobei die Membran eine expandierte Polytetrafluorethylenschicht (14) auf einer Seite und eine Trägerschicht (16) aus hydrophobem Material auf der anderen Seite aufweist und die Membran mindestens teilweise über dem Filter mit der Trägerschicht (16) zum Filter weisend liegt, und **gekennzeichnet durch**
c. einer Verbindungsfolie (12) mit einem Innen- und einem Außenende, wobei die Verbindungsfolie (12) an ihrem Innenende mit der Polytetrafluorethylenschicht (14) und an ihrem Außenende mit dem nichtkörperseitigen Element (4) verschweißt ist.

2. Filteranordnung nach Anspruch 1, wobei die Trägerschicht PET (Polyester) ist.

3. Filteranordnung nach Anspruch 1 oder 2, wobei die mit der Membranschicht verschweißte Folie mindestens 75 Mikrometer dick ist und thermische Kunststoffeigenschaften hat, die für Schweißen bei hoher Temperatur und hohem Druck geeignet sind.

4. Filteranordnung nach Anspruch 1, 2 oder 3, wobei das geruchsbeseitigende Material Aktivkohle ist.

5. Filteranordnung nach einem der vorstehenden Ansprüche, wobei die mikroporöse Verbundmembran ein Laminat ist.

6. Filteranordnung nach einem der vorstehenden Ansprüche, wobei das Filter mit dem nichtkörperseitigen Element auf der Innenseite des Stomabeutels verschweißt ist.

## Revendications

1. Ensemble de filtre pour la fixation à une poche de stomie, la poche de stomie étant formée à partir d'un panneau côté corps et un panneau non côté corps reliés ensemble, comprenant :
a. un filtre poreux (10) contenant un matériau désodorisant pour désodoriser le gaz passant à travers celui-ci, ledit filtre étant fixé au panneau non côté corps (4), le panneau non côté corps ayant une sortie (30) pour évacuer le gaz désodorisant à travers celle-ci à l'extérieur de la poche,
b. une membrane microporeuse composite à deux côtés (8) couvrant au moins partiellement ledit filtre pour obstruer l'entrée de liquides dans le filtre, ladite membrane étant constituée d'une couche de polytétrafluoroéthylène expansée (14) sur un côté et d'une couche de support (16) en matériau hydrophobe sur l'autre côté, ladite membrane couvrant au moins partiellement ledit filtre avec ladite couche de support étant située face audit filtre, et **caractérisé par**
c. un film de connexion (12) ayant des extrémités intérieure et extérieure, ledit film de connexion (12) étant soudé au niveau de son extrémité intérieure à ladite couche de polytétrafluoroéthylène (14) et au niveau de son extrémité extérieure audit panneau non côté corps (4).

2. Ensemble de filtre selon la revendication 1, dans lequel ladite couche de support est réalisée en PET (Polyester).

3. Ensemble de filtre selon la revendication 1 ou 2, dans lequel ledit film soudé à la couche de membrane présente une épaisseur supérieure ou égale à 75 microns et présente des propriétés plastiques thermiques convenables au soudage à haute température et à haute pression.

4. Ensemble de filtre selon la revendication 1, 2 ou 3, dans lequel ledit matériau désodorisant est du charbon actif.

5. Ensemble de filtre selon l'une des revendications précédentes, dans lequel ladite membrane microporeuse composite est un stratifié.

6. Ensemble de filtre selon l'une des revendications précédentes, dans lequel ledit filtre est soudé au panneau non côté corps à l'intérieur de la poche de stomie.
